## Europäisches Patentamt
## European Patent Office
## Office européen des brevets

(19)

(11) Veröffentlichungsnummer: **0 063 320**
A1

(12) # EUROPÄISCHE PATENTANMELDUNG

(21) Anmeldenummer: **82103012.9**

(22) Anmeldetag: **08.04.82**

(51) Int. Cl.³: **C 07 C 69/145**
**C 07 C 67/055**

(30) Priorität: **18.04.81 DE 3115647**

(43) Veröffentlichungstag der Anmeldung:
**27.10.82 Patentblatt 82/43**

(84) Benannte Vertragsstaaten:
**BE DE FR GB IT NL**

(71) Anmelder: **BASF Aktiengesellschaft**
**Carl-Bosch-Strasse 38**
**D-6700 Ludwigshafen(DE)**

(72) Erfinder: **Weitz, Hans-Martin, Dr.**
**Auf dem Koeppel 40**
**D-6702 Bad Duerkheim(DE)**

(72) Erfinder: **Schnabel, Rolf, Dr.**
**Tilsiter Strasse 9**
**D-6707 Schifferstadt(DE)**

(72) Erfinder: **Fischer, Rolf, Dr.**
**Bergstrasse 98**
**D-6900 Heidelberg(DE)**

(54) **Verfahren zur Herstellung von Acyloxybutenen.**

(57) Verfahren zur Herstellung von Acyloxybutenen durch Umsetzung von Buta-1,3-dienen mit Sauerstoff und niedermolekularen Carbonsäuren in Gegenwart eines Palladium oder Platin sowie Antimon und/oder Bismut enthaltenden Trägerkatalysators, der als zusätzliches Element Kupfer enthält.

EP 0 063 320 A1

BASF Aktiengesellschaft                    O.Z. 0050/035101

Verfahren zur Herstellung von Acyloxybutenen

Die Erfindung betrifft eine Verbesserung des Verfahrens zur Herstellung von Acyloxybutenen wie 1,2- und 1,4-Acyloxybutenen durch Umsetzung von ggf. substituiertem Buta-1,3-dien mit Sauerstoff und niedermolekularen Carbonsäuren in Gegenwart eines Palladium oder Platin sowie Antimon und/oder Bismut enthaltenden Trägerkatalysators.

Bekanntlich lassen sich Buta-1,3-diene mit Sauerstoff und Carbonsäuren in Gegenwart von gelösten Palladium- und Kupfersalzen zu Buten-1,2- und 1,4-diolestern umsetzen. Nach den Angaben der GB-PS 1 138 366 erhält man durch Umsetzung von Piperylen mit Luft in Gegenwart von in Eisessig gelöstem Palladiumacetat, Kupferacetat und Lithiumacetat Gemische aus 1,4-Diacetoxy-pent-2-en und 4,5-Diacetoxy-pent-2-en (Molverhältnis 4 : 1). In ähnlicher Weise wird Butadien nach der GB-PS 1 277 837 mit Sauerstoff in Gegenwart von in Eisessig gelöstem Palladiumacetat, Kupferacetat und Kaliumacetat zu 1,4-Diacetoxy-but-2-en umgesetzt. Eine Variante dieser Katalysatorkombination wird in der japanischen Patentanmeldung 19 293 (1973) genannt, in der angegeben ist, daß die Ausbeute durch Zugabe von Acetanhydrid erhöht werden kann. In der japanischen Patentanmeldung 13714 (1976) werden für die Umsetzung von Butadien mit Essigsäure und Sauerstoff Lösungen von Palladiumacetat, Kupferacetat und Lithiumchlorid vorgeschlagen.

Es ist weiterhin schon vorgeschlagen worden, die Umsetzung von Butadien mit Essigsäure und Sauerstoff ("Acetoxylierung") in Gegenwart von metallisches Palladium enthaltenden festen Katalysatoren mit Aktivkohle als Träger durchzuführen, wobei diese Katalysatoren in Lösungen von Kupfer-

Hee/P

acetat und Lithiumacetat in Eisessig und Acetanhydrid (japanische Patentanmeldung 126 607 (1975)) von Kupferchlorid, Lithiumacetat und Antimontrichlorid (japanische Patentanmeldung 39 004 (1972)) oder von Kupferchlorid in Acetanhydrid (japanische Patentanmeldung 39 003 (1972)) suspendiert wurden. Bei der in der DE-PS 20 12 903 beschriebenen Acetoxylierung von Butadien werden metallisches Palladium enthaltende Katalysatoren verwendet, bei deren Herstellung Kupfersalze wie Kupferchlorid oder Kupferacetat zugegeben werden.

Einer technischen Nutzung dieser Verfahren steht entgegen, daß die Katalysatoren nur einen niedrigen Butadien-Umsatz bewirken und mit geringer Selektivität arbeiten. Weiterhin ist die Verwendung des teuren Acetanhydrids von Nachteil, auch ist es schwierig, gewisse homogen gelöste Katalysatorkomponenten abzutrennen, gegebenenfalls aufzuarbeiten und in den Prozeß zurückzuführen.

In der DE-PS 22 17 452 wird ein Verfahren zur Acetoxylierung von Butadien beschrieben, bei dem man Palladium-Katalysatoren verwendet, die zusätzlich Antimon, Wismut, Tellur oder Selen enthalten. Diese Selen und/oder Tellur enthaltenden Katalysatoren haben bei ihrer praktischen Anwendung den Nachteil, daß sie wegen der hohen Toxizität der Selen- und Tellurverbindungen (z.B. MAK-Werte von 0,1 $mg/m^3$, berechnet auf das Element) bei ihrer Herstellung und Handhabung sehr aufwendige Sicherheitsmaßnahmen erforderlich machen. Die Palladium und Antimon sowie die Palladium und Bismut enthaltenden Katalysatoren zeigen eine unbefriedigende katalytische Aktivität. Ferner sind aus der DE-OS 28 20 519 Katalysatoren für den genannten Zweck bekannt, die neben Platin bzw. Palladium noch Kupfer enthalten, jedoch können auch diese Katalysatoren nicht voll befriedigen. Ebenso verhält es sich mit den Trägerkatalysatoren der älteren Anmeldung P 29 43 407.9, die als weiteres Element neben Palladium oder Platin sowie neben Kupfer noch Tellur enthalten.

Es wurde nun gefunden, daß man bei der Herstellung von Acyloxybutenen durch Umsetzung von ggf. substituiertem Buta-1,3-dien mit Sauerstoff und niedermolekularen Carbonsäuren in Gegenwart eines Palladium oder Platin sowie Antimon und/oder Bismut enthaltenden Trägerkatalysators besonders vorteilhafte Ergebnisse erzielt, wenn der Katalysator als zusätzliches Element Kupfer enthält.

Die neuen Katalysatoren liefern besonders vorteilhafte Resultate, wenn sie intermetallische Palladium-Kupfer- oder Platin-Kupfer-Verbindungen enthalten.

Nach dem neuen Verfahren erhält man hohe Raum-Zeit-Ausbeuten. Die nach dem erfindungsgemäßen Verfahren zu verwendenden Katalysatoren erweisen sich als hochselektiv in Bezug auf die erwünschte Bildung von Butendioldiacetaten.

Die nach der Erfindung zu verwendenden Trägerkatalysatoren enthalten als aktive Bestandteile, die auf dem Trägermaterial aufgebracht sind, Palladium oder Platin, Antimon und/oder Bismut sowie außerdem Kupfer.

Die besonders vorteilhaften Katalysatoren dieser Art enthalten eine intermetallische Palladium-Kupfer- oder Platin-Kupfer-Verbindung. Solche intermetallischen Phasen, die die Zusammensetzung $PdCu_3$, $PtCu_3$ oder $PdCu$, $PtCu$ haben, sind röntgengraphisch nachweisbar. Intermetallische Phasen aus Palladium oder Platin einerseits und Antimon und/oder Bismut andererseits wurden bei der Röntgenanalyse hingegen nicht beobachtet.

Die Katalysatoren der genannten Art enthalten beispielsweise (bezogen auf das Katalysatorgewicht) 1 bis 10 % Palladium oder Platin, 0,1 bis 30 % Kupfer und 0,01 bis 10 % Antimon und/oder Bismut. Bevorzugt ist die Verwendung von Trägerkatalysatoren, die je Grammatom Palladium oder Platin 0,01 bis 6, vorzugsweise 1 bis 3,5 Grammatom Kupfer und 0,01 bis 1, vorzugsweise 0,01 bis 0,4 Grammatom Antimon und/oder Bismut enthalten, wobei im Falle des Antimons die bevorzugte untere Grenze 0,04 Grammatom ist.

Die Gesamtmenge der auf den Träger aufgebrachten katalytisch wirksamen Metalle beträgt im allgemeinen, bezogen auf den Trägerkatalysator, zweckmäßig z.B. 0,01 bis 50, vorzugsweise 1 bis 30 Gew.%. Die Entscheidung, welche Menge die günstigste ist, kann auch von wirtschaftlichen Überlegungen bestimmt werden.

Als Trägermaterialien kommen für die Katalysatoren z.B. Aktivkohle, Bauxit, Bimsstein, Kieselgel, Kieselgur oder andere Formen der Kieselsäure, Magnesia, Ton und Tonerde in Betracht. Die Träger können durch eine übliche Vorbehandlung, z.B. mit einer Säure, für ihre Zweckbestimmung verbessert werden.

Für die Herstellung des Katalysators ist die zu wählende Palladium- oder Platin-Verbindung nicht entscheidend. So kann man z.B. Palladium- oder Platin-halogenide wie Palladium- oder Platinchlorid, ein Salz einer organischen Säure wie Palladiumacetat oder Platinacetat oder auch die Nitrate oder Oxide verwenden. Man kann aber auch von anderen Verbindungen, insbesondere Komplexverbindungen, ausgehen. Auch Kupfer, Antimon und Bismut können weitgehend in Form frei wählbarer Verbindungen in die herzustellenden Katalysatoren eingeführt werden. Aus Zweck- mäßigkeitsgründen wird man zu löslichen Verbindungen greifen.

Die Katalysatoren werden z.B. hergestellt, indem man den Träger in einer Lösung dispergiert, die eine Palladium- oder Platinverbindung sowie eine Kupfer- und Antimon- und/oder Bismut-Verbindung enthält, das Lösungsmittel verdampft und den Rückstand in einem Gasstrom aus z.B. Wasserstoff oder Stickstoff, der mit einer reduzierenden Verbindung wie Hydrazin, Methanol oder Formaldehyd beladen ist, bei 100 bis 400°C reduziert. Die Reduktion des getrockneten Katalysators kann auch mit flüssigen Reduktionsmitteln geschehen. Man kann den Katalysator auch her-stellen, indem man Träger und Lösung gemeinsam mit einem z.B. alkalischen Fällungsmittel behandelt, den Niederschlag abtrennt und die Reduktion anschließend vornimmt. Eine sehr brauchbare Methode zur Herstellung der Katalysatoren ist die Fällung der Metalle aus wäßrigen Salzlösungen durch Reduktionsmittel wie Formaldehyd und Hydrazin bei einem bestimmten pH-Wert (siehe z.B. J. Am. Chem. Soc. 83 (1961) S. 4916).

Zweckmäßig werden die so erhaltenen Katalysatoren noch in einem reduzierend wirkenden Gasstrom auf höhere Temperaturen erhitzt.

Palladium oder Platin, Kupfer und Antimon und/oder Bismut können gleichzeitig oder in beliebiger Reihenfolge nacheinander auf dem Träger abgeschieden werden; in manchen Fällen kann der Träger in Form einer löslichen Verbindung zugefügt und gemeinsam mit dem wirksamen Metall ausgefällt werden.

Man kann jedes Reduktionsverfahren anwenden, durch welches die verwendeten Elemente in den metallischen Zustand überführt werden.

Es kann zweckmäßig sein, die nach diesen an sich bekannten Verfahren erhältlichen Katalysatoren, bei denen die Metalle in Form zufälliger physikalischer Mischungen vorliegen, in die Form bringen, die eine intermetallische Palladium-Kupfer- oder Platin-Kupfer-Verbindung enthält.

Intermetallische Phasen, an denen Palladium oder Platin beteiligt sind, sind bekannt. Sie haben eine kubisch-flächenzentrierte, kubisch-innenzentrierte oder auch tetragonale Struktur, wobei auch sogenannte Überstrukturen auftreten können. Solche Phasen werden z.B. in Gmelins Handbuch der Anorganischen Chemie, 8. Aufl., Bd. 68, Teil A, S. 617-652 (1951), beschrieben.

Zweckmäßig werden die Katalysatoren vor ihrer Verwendung auf 400 bis 900°C, erhitzt. Da sich beim Erhitzen schließlich eine gewisse Stabilisierung des einmal erreichten katalytisch wirksamen Zustandes einstellt, ist die Anwendung einer bestimmten Höchstzeit im allgemeinen nicht erforderlich. Falls der Träger oder die Metalle unter den Bedingungen des Erhitzens zur Oxidation neigen, wird man zweckmäßig das Erhitzen in Gegenwart einer reduzierenden Atmosphäre, z.B. in reinem Wasserstoff, oder in Gegenwart eines Inertgases wie Stickstoff vornehmen.

Als ggf. substituiertes Butadien kann man für das erfindungsgemäße Verfahren außer Butadien auch andere konjugierte Diolefine wie Isopren, 2,3-Dimethyl-buta-1,3-dien, Piperylen oder durch Acyloxygruppen substituierte Buta--1,3-dien verwenden. Die genannten Diolefine können für sich oder als Mischungen, die z.B. auch noch andere Kohlenwasserstoffe wie Monoolefine und Paraffinkohlenwasserstoffe enthalten können, eingesetzt werden. Solche Mischungen stehen z.B. als sogenannte $C_4$-Schnitte zur Verfügung. Als niedermolekulare Carbonsäuren kommen z.B. Fettsäuren mit 1 bis 3 C-Atomen, wie Ameisensäure, Essigsäure oder Propionsäure in Betracht.

Die Umsetzung zur Herstellung der Acyloxybutene nimmt man in der Gas- oder Flüssigphase bei Temperaturen von 70 bis $180^{\circ}$C vor. In der Gasphase arbeitet man vorzugsweise bei 120 bis $150^{\circ}$C und in der Flüssigphase vorzugsweise bei 70 bis $110^{\circ}$C. Der Reaktionsdruck ist durch die Verfahrensweise gegeben und kann zwischen atmosphärischem Druck und z.B. 100 bar betragen. Das Verfahren kann chargenweise oder kontinuierlich z.B. mit fixiertem Bett, Wirbelbett oder Dreiphasenfließbett durchgeführt werden.

Die nach dem Verfahren der Erfindung herstellbaren Acyloxybutene sind wertvolle Zwischenprodukte, z.B. für die Herstellung von But-2-en-1,4-diol, Butan-1,4-diol und Tetrahydrofuran. Das in untergeordneten Mengen gebildete Buten-3,4-diol-diacetat (Vinylglykoldiacetat) ist als Zwischenprodukt zur Herstellung von Vitaminen und anderen biologisch wirksamen Verbindungen geeignet. Die bei der Acetoxylierung von Isopren oder 1-Acetoxy-2-methyl-butadien erhaltenen 2-Methyl-1,4-diacetoxybut-2-ene oder 1,1,4-Triacetoxy-2-methyl-but-2-ene sind wertvolle Zwischenprodukte z.B. für die Synthese von Terpenverbindungen.

Bei dem erfindungsgemäßen Verfahren werden die gewünschten Acyloxybutene in hoher Ausbeute gebildet. Die Vergleichsbeispiele 1 und 2 zeigen, daß Palladium-Bismut- ebenso wie Palladium-Kupferkatalysatoren schlechtere Aktivitäten als Palladium-Kupfer-Bismutkatalysatoren aufweisen. Vorteilhaft ist weiterhin, daß Antimon und vor allem Bismut wesentlich weniger toxisch als Selen und Tellur sind, und daß die Gegenwart von Antimon bzw. Bismut die Ausbildung der häufig erwünschten intermetallischen Pd/Cu- bzw. Pt/Cu-Phase beschleunigt.

Im experimentellen Teil werden folgende Abkürzungen benutzt:

trans-1,4-Diacetoxy-but-2-en = trans-1,- DAB
cis-1,4-Diacetoxy-but-2-en   = cis-1,4-DAB
3,4-Diacetoxy-but-1-en       = 3,4-DAB
Gesamtmenge der vorgenannten Isomeren = DAB

Bei den Prozentangaben handelt es sich um Gewichtsprozente. Die Katalysatoraktivität ist als g DAB-Ausbeute pro kg Katalysator pro Stunde definiert.

Beispiel 1

a)    Herstellung eines Pd/Cu/Bi-Katalysators

Eine Lösung von 7,2 g Kupferpulver in 80 cm$^3$ 3 n Salpetersäure wurde bei Raumtemperatur mit einer Lösung von 8,3 g PdCl$_2$ in 40 cm$^3$ eines warmen Gemisches aus 66%iger Salpetersäure in 32%iger Salzsäure (Volumenverhältnis 1:1) und einer Lösung von 2,3 g Bi(NO$_3$)$_5$.5H$_2$O in 100 cm warmer 3 n Salpetersäure vermischt. Sodann wurden 100 g Aktivkohle (Teilchengröße 0,3 bis 0,5 mm; Oberfläche 1100 m$^2$/g) vorgelegt, die zuvor bei 70°C 5 Stunden lang mit 15%iger Salpetersäure gerührt, nach Filtration neutral gewaschen und bei 150°C unter Vakuum getrocknet worden waren (Schwefelgehalt 0,21 %) wonach die vereinigte Metallsalzlösung hinzugegeben wurde. Anschließend wurde soviel Wasser hinzugefügt, daß die Kohle völlig benetzt war. Dann wurde am Rotationsverdampfer bei $85^{o}$C und Wasserstrahlunterdruck zur Trockene eingedampft. Der Katalysator wurde anschließend 16 Stunden bei $150^{o}$C, dann 8 Stunden bei $400^{o}$C mit bei Raumtemperatur mit Methanol gesättigtem Stickstoff aktiviert, wonach er unter strömendem Stickstoff auf Raumtemperatur abgekühlt wurde.

Nach der Elementaranalyse enthielt der Katalysator 5,0 % Palladium, 9,0 % Kupfer und 1,0 % Bismut. Das Grammatomverhältnis von Pd:Cu:Bi betrug 1:3,02:0,10. Überwiegend lag das Pd in intermetallischer Phase mit Cu vor.

b)   Acetoxylierung von Butadien (Autoklavenversuch unter Druck)

3 g des nach Beispiel (1a) hergestellten Katalysators wurden zusammen mit 30 cm$^3$ Eisessig und 3 cm$^3$ Butadien in einen mit einem Magnetrührer versehenen 50 ml-Autoklaven gegeben. Der verschlossene Autoklav wurde auf $95^{o}$C aufgeheizt, der Rührmotor auf 150 U/min eingestellt und unter 20 bar Sauerstoffdruck gesetzt. Nach 20 min Reaktionszeit wurde entspannt und abgekühlt. Der flüssige Reaktionsaustrag enthielt neben nicht umgesetzter Essigsäure und nicht umgesetztem Butadien 3,71 % trans-1,4-DAB, 0,70 % cis-1,4-DAB und 0,74 3,4-DAB. Das entspricht einer Katalysatoraktivität von 1550 g DAB/kg.h.

## Vergleichsbeispiel 1

Herstellung und Verwendung eines Pd/Bi-Katalysators

Analog Beispiel (1a) wurde ein Trägerkatalysator hergestellt, der 5,0 % Pd und 1,0 % Bi, jedoch kein Cu enthielt. Mit diesem Katalysator wurde unter den Versuchsbedingungen von Beispiel (1b) ein flüssiger Reaktionsaustrag erhalten, der 0,25 % trans-1,4-DAB, 0,05 % cis-1,4-DAB und 0,05 3,4-DAB enthielt. Dies entspricht einer Katalysatoraktivität von 105 g DAB/kg.h.

## Vergleichsbeispiel 2

Herstellung und Verwendung eines Pd/Cu-Katalysators

Analog Beispiel (1a) wurde ein Trägerkatalysator hergestellt, der 5,0 % Pd und 9,0 % Cu, jedoch kein Bi enthielt. Mit diesem Katalysator wurde unter den Versuchsbdingungen von Beispiel (1b) ein flüssiger Reaktionsaustrag erhalten, der 1,30 % trans-1,4-DAB, 0,33 % cis-1,4-DAB und 0,17 % 3,4-DAB enthielt. Dies entspricht einer Katalysatoraktivität von 540 g DAB/kg.h.

## Beispiel 2

Herstellung und Verwendung eines Pd/Cu/Sb-Katalysators

Auf die in Beispiel (1a) beschriebene Weise wurde ein Trägerkatalysator hergestellt, der anstelle des Bismuts die gleiche Gewichtsmenge Antimon enthielt. Mit diesem Katalysator wurde unter den Bedingungen von Beispiel (1b) ein Reaktionsaustrag erhalten, der 8,66 % trans-1,4-DAB, 1,84 % cis-1,4-DAB und 1,55 % 3,4-DAB enthielt. Dies entspricht einer Katalysatoraktivität von 3980 g DAB/kg.h.

0063320

Die folgende Übersicht veranschaulicht die nach den vorstehenden Beispielen erzielten Ergebnisse.

| | Metallgehalte des Katalysators (Gew.-%) | | | | Katalysatoraktivität für die Herstellung von DAB (g/kg.h) |
|---|---|---|---|---|---|
| | Pd | Cu | Bi | Sb | |
| Beispiel 1 | 5 | 9 | 1 | – | 1550 |
| Beispiel 2 | 5 | 9 | – | 1 | 3980 |
| Vergl.-Bsp. 1 | 5 | – | 1 | – | 105 |
| Vergl.-Bsp. 2 | 5 | 9 | – | – | 540 |

Patentansprüche

1. Verfahren zur Herstellung von Acyloxybutenen durch Umsetzung von ggf. substituiertem Buta-1,3-dien mit Sauerstoff und niedermolekularen Carbonsäuren in Gegenwart eines Palladium oder Platin sowie Antimon und/oder Bismut enthaltenden Trägerkatalysators, dadurch gekennzeichnet, daß der Katalysator als zusätzliches Element Kupfer enthält.

2. Verfahren nach Anspruch 1, dadurch gekennzeichnet, daß der Katalysator eine intermetallische Palladium-Kupfer-Verbindung enthält.

3. Verfahren nach Anspruch 1, dadurch gekennzeichnet, daß der Katalysator eine intermetallische Platin--Kupfer-Verbindung enthält.

4. Verfahren nach Anspruch 1, dadurch gekennzeichnet, daß man einen Trägerkatalysator verwendet, der, bezogen auf das Katalysatorgewicht, 1 bis 10 % Palladium oder Platin, 0,1 bis 30 % Kupfer und 0,01 bis 10 % Antimon und/oder Bismut enthält.

5. Verfahren nach Anspruch 1, dadurch gekennzeichnet, daß man einen Trägerkatalysator verwendet, der je Grammatom Palladium oder Platin 0,01 bis 6 Grammatome Kupfer und 0,01 bis 1 Grammatom Antimon und/oder Bismut enthält.

6.  Verfahren nach Anspruch 1, dadurch gekennzeichnet, daß man einen Trägerkatalysator verwendet, der je Grammatom Palladium oder Platin 1 bis 3,5 Grammatome Kupfer und 0,01 bis 0,4 Grammatom Antimon und/oder Bismut enthält, wobei im Falle des Antimons die bevorzugte untere Grenze 0,04 Grammatom ist.

7.  Verfahren nach Anspruch 1, dadurch gekennzeichnet, daß man als gegebenenfalls substituiertes Buta-1,3-dien das nicht substituierte Buta-1,3-dien, Isopren oder 1-Acetoxy-2-methyl-buta-1,3-dien verwendet.

0063320

Nummer der Anmeldung

EP 82 10 3012

Europäisches Patentamt

**EUROPÄISCHER RECHERCHENBERICHT**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| X | DE-A-2 723 961 (MITSUBISHI)<br>* Seite 4; Seite 5, Abschnitt 1 *<br><br>--- | 1,4 | C 07 C 69/145<br>C 07 C 67/055 |
| Y | FR-A-1 318 208 (HOECHST)<br>* Seite 2, linke Spalte, Abschnitt 3,4; rechte Spalte, Abschnitt 3 *<br><br>----- | 1 | |

RECHERCHIERTE SACHGEBIETE (Int. Cl. 3)

C 07 C 69/00
C 07 C 67/00

Der vorliegende Recherchenbericht wurde für alle Patentansprüche erstellt.

| Recherchenort<br>DEN HAAG | Abschlußdatum der Recherche<br>02-07-1982 | Prüfer<br>KINZINGER J.M. |
|---|---|---|